# EUROPÄISCHE PATENTSCHRIFT

(11) **EP 3 795 116 B1**
(45) Veröffentlichungstag und Bekanntmachung des Hinweises auf die Patenterteilung: **27.10.2021**
(21) Anmeldenummer: 20190635.1
(22) Anmeldetag: 12.08.2020
(51) Int. Cl.: A61F 2/06, A61L 27/18, A61L 27/50

(54) **GEFÄSSPROTHESE**
VASCULAR PROSTHESIS
PROTHÈSE VASCULAIRE

(30) Priorität: 20.09.2019 DE 102019125367
(43) Veröffentlichungstag der Anmeldung: 24.03.2021
(73) Patentinhaber: E.S. Bio-Tech Limited, 4105 Limassol (CY)
(72) Erfinder: Heinsch, Manfred, 61137 Schöneck (DE); Doss, Mirko, 60599 Frankfurt am Main (DE); Brüning, Martin, 73061 Ebersbach (DE); Müller, Eberhard, 70565 Stuttgart (DE); Ullmann, Michael, 71229 Leonberg (DE)
(74) Vertreter: Wagner Albiger & Partner Patentanwälte mbB

(56) Entgegenhaltungen:
- EP-A1- 2 319 454
- EP-A1- 2 574 305
- DE-A1-102006 028 221

## Beschreibung

Die Erfindung betrifft eine Gefäßprothese zum Anschluss an ein natürliches kardiovaskuläres System, umfassend eine Volumenkammer, wobei die Volumenkammer in einem Blutdruckbereich unterhalb eines mindestens 100 mm Hg betragenden Druckschwellenwertes D ein Druck-Dehnungsverhalten aufweist, welches im Wesentlichen dem Druck-Dehnungsverhalten eines natürlichen Blutgefäßes entspricht, während sich das Volumen der Volumenkammer in einem Blutdruckbereich oberhalb des Druckschwellenwertes druckabhängig um mindestens 10 cm³ vergrößert. Die Erfindung betrifft auch ein Verfahren zum Herstellen einer solchen Gefäßprothese.

Der Ersatz erkrankter Abschnitte der Aorta durch Gefäßprothesen aus Kunststoff in Form eines Interponats bzw. die Umgehung erkrankter Abschnitte der Aorta in Form eines Bypasses sind seit Langem etablierte chirurgische Verfahren. Die dabei verwendeten Prothesen werden üblicherweise als textile Rohrprothesen aus vom Körper nichtresorbierbaren synthetischen Polymeren wie z. B. Polytetrafluorethylen (PTFE) oder Polyester wie Polyethylenterephthalat (PET) durch Weben, Wirken oder Stricken hergestellt. Gegebenenfalls können solche Gefäßprothesen auch verzweigt ausgebildet sein als sogenannte Y-Prothesen.

Die heute zum Einsatz kommenden Gefäßprothesen sind zwar durch Maßnahmen wie Plissieren bzw. Crimping in geringem Maße längs- und querdehnbar, sie besitzen aber nicht die elastischen Gewebeeigenschaften der natürlichen Aorta bzw. der natürlichen Arterien. Insbesondere sind sie nicht ausreichend in der Lage, die Windkesselfunktion der natürlichen Gefäße nachzubilden. Unter der Windkesselfunktion versteht man das kurzzeitige Zurückhalten eines Teils des während der Systole vom Herzen ausgeworfenen Blutvolumens in den elastischen Arterien und dessen kontinuierliche Abgabe während der Diastole, wodurch der Blutstrom insgesamt vergleichmäßigt und der Druckunterschied zwischen Systole und Diastole verringert wird. Dabei wird die kurzzeitige Speicherung und die nachfolgende Abgabe des Blutvolumens maßgeblich durch die elastischen Eigenschaften der natürlichen Gefäßwände bewirkt, die nach einer während der Systole erfolgten geringfügigen Ausdehnung während der Diastole wieder in ihren Ausgangszustand zurückstreben. Diese Eigenschaft können bekannte Gefäßprothesen bislang nur unzureichend nachbilden.

Die fehlende Elastizität der Gefäßprothesen wirkt sich bei der bestimmungsentsprechenden Langzeitanwendung im menschlichen Körper negativ auf Herz, Aorta und Gefäßprothese selbst aus. Nach Ersatz des erkrankten Aortensegments durch Interposition einer nichtelastischen Gefäßprothese kommt es zu einem Missverhältnis zwischen den Volumenkapazitätseigenschaften der verbleibenden natürlichen Aorta und der implantierten steiferen Gefäßprothese. Dieses Missverhältnis wird auch als Compliance-Missverhältnis bezeichnet. Dabei beeinträchtigt die fehlende Elastizität einer im Bereich der herznahen Aorta ascendens implantierten Gefäßprothese die Funktion der Aortenklappe, sofern diese beim Aortenersatz erhalten wurde, so dass die Aortenklappe undicht werden kann. Zudem wird der Blutdruck durch die fehlende Elastizität in Amplitudenhöhe und -form ungünstig verändert. Durch Wegfall bzw. Beeinträchtigung der Windkesselfunktion der Aorta kommt es insgesamt zu ungünstigen hämodynamischen Veränderungen mit einer Zunahme der Pulswellengeschwindigkeit und einem steileren Anstieg der Blutdruckkurve bei insgesamt vergrößerter Blutdruckamplitude.

Diese aufgrund des Compliance-Missverhältnisses sich einstellenden ungünstigen Blutdruckveränderungen stellen eine andauernde Belastung für das Herz da, die zu einer muskulären Hypertrophie (Herzwandverdickung) und einer zunehmenden Funktionsstörung in Form einer Herzinsuffizienz (Herzschwäche) führen kann. Zudem wirkt sich die vermehrte Belastung durch die Blutdruckänderungen negativ auf die verbliebene natürliche Aorta und deren Verbindungsstellen mit der implantierten Gefäßprothese aus. Hier kann es zur Bildung eines sogenannten Anschlussaneurysmas, d. h. zu einer Erweiterung bzw. Aussackung im Verbindungsbereich von Prothese und Aorta, kommen, das unter Umständen operativ beseitigt werden muss. Die sich unmittelbar nach Prothesenimplantation einstellende unphysiologische Blutdruckbelastung kann zudem zu einer mitunter erheblichen und nicht reversiblen Zunahme des Prothesendurchmessers führen, was in der Folge beobachtet und regelmäßig kontrolliert werden muss. Dies geschieht üblicherweise durch jährliche CT-Untersuchungen, die wiederum mit erhöhter Strahlenbelastung einhergehen. Bei sich abzeichnender Gefahr des Einreißens der Gefäßprothese oder der Verbindungsstelle muss die Gefäßprothese operativ ausgetauscht werden, was mit einem deutlich erhöhten Operationsrisiko verbunden ist.

Es sind bereits Gefäßprothesen bekannt, die aufgrund ihrer speziellen konstruktiven Ausgestaltung zumindest teilweise in der Lage sind, die natürliche Windkesselfunktion nachzubilden. So beschreibt die EP 2 574 305 B1 ein Ausgleichsgefäß zur Beeinflussung des Blutdrucks, umfassend eine Volumenkammer sowie Anschlussmittel zum Anschluss der Volumenkammer an ein natürliches kardiovaskuläres System, wobei durch eine Druckänderung in dem kardiovaskulären System eine Volumenänderung der Volumenkammer bewirkbar ist, sowie Anpassungsmittel, welche die Volumenänderung der Volumenkammer in einem unteren Druckbereich unterhalb eines mindestens 100 mm Hg betragenden Druckschwellenwertes auf maximal 10 cm³ begrenzen und welche in einem oberen Druckbereich zwischen dem Druckschwellenwert und 150 mm Hg eine Volumenänderung der Volumenkammer von mindestens 10 cm³ bewirken. Die Anpassungsmittel umfassen dabei einen Rahmen sowie mindestens einen mit diesem zusammenwirkenden Federkörper, wobei Rahmen und Federkörper außerhalb der Volumenkammer angeordnet sind und wobei der Rahmen zwei Stirnteile sowie mindestens drei die beiden Stirnteile verbindende Stützstäbe aufweist, zwischen denen sich der mindestens eine Federkörper erstreckt. Der Aufbau des Ausgleichsgefäßes ist vergleichsweise aufwendig und kompliziert. Zudem kann das Ausgleichsgefäß gemäß EP 2 574 305 B1 nicht minimalinvasiv implantiert werden. Beides erscheint verbesserungswürdig. Darüber hinaus wird eine Verbesserung hinsichtlich des Einwachsverhaltens der Gefäßprothese angestrebt.

Die DE 10 2006 028 221 A1 offenbart eine schlauchförmige Gefäßprothese, deren Wandung eine elastische Vliesstruktur aufweist. Die Vliesstruktur weist durch Begrenzung der Dehnbarkeit ein Druck-Dehnungsverhalten auf, das im Wesentlichen der Dehnbarkeit von natürlichen Arterien entspricht.

In der EP 2 319 454 A1 ist eine Bypass-Vorrichtung zur Beeinflussung des Blutdrucks beschrieben, welche ein Implantat mit einer Volumenkammer sowie Anpassungsmittel zum Anschluss an ein natürliches kardiovaskuläres System umfasst. Durch die Anpassungsmittel wird eine Volumenänderung eines Volumens der Volumenkammer bei einer Druckänderung in dem kardiovaskulären System oder in der Volumenkammer ermöglicht oder bewirkt.

Die Aufgabe der vorliegenden Erfindung ist es, eine Gefäßprothese bereitzustellen, deren Eigenschaften in höherem Maße als bei aus dem Stand der Technik bekannten Gefäßprothesen mit den Eigenschaften natürlicher Blutgefäße übereinstimmen und welche gleichzeitig einen einfachen Aufbau aufweist und minimalinvasiv implantierbar ist. Es ist eine weitere Aufgabe der Erfindung, ein Verfahren zur Herstellung einer solchen Gefäßprothese anzugeben.

Diese Aufgaben werden gelöst durch eine Gefäßprothese mit den Merkmalen des Patentanspruchs 1 sowie durch ein Verfahren zur Herstellung einer Gefäßprothese mit den Merkmalen des Patentanspruchs 16.

Vorteilhafte Ausgestaltungen und Weiterbildungen der Erfindung sind Gegenstand der abhängigen Ansprüche.

Gemäß Patentanspruch 1 handelt es sich bei der Erfindung um eine Gefäßprothese zum Anschluss an ein natürliches kardiovaskuläres System, umfassend eine Volumenkammer, wobei die Volumenkammer in einem Blutdruckbereich unterhalb eines mindestens 100 mm Hg betragenden Druckschwellenwertes D ein Druck-Dehnungsverhalten aufweist, welches im Wesentlichen dem Druck-Dehnungsverhalten eines natürlichen Blutgefäßes entspricht, während sich das Volumen der Volumenkammer in einem Blutdruckbereich oberhalb des Druckschwellenwertes D druckabhängig um mindestens 10 cm³ vergrößert. Die erfindungsgemäße Gefäßprothese zeichnet sich dadurch aus, dass sie als textiler Schlauch ausgebildet ist, wobei der textile Schlauch im Bereich der Volumenkammer ein elastisches Garn umfasst, welches einen Kern aus Silikongarn aufweist, der mit einem Garn aus Polyethylenterephthalat (PET) umwunden ist.

Mit anderen Worten handelt es sich bei der Erfindung um eine textile, schlauchförmige Gefäßprothese, deren Volumen sich ab einem definierten, mindestens 100 mm Hg betragenden Druckschwellenwert D durch radiale Dehnung im elastischen Bereich vergrößert und die damit ein definiertes zusätzliches Volumen zur Verfügung stellt, wobei die Volumenänderung bzw. die radiale Dehnung weitestgehend reversibel ist und sich somit das ursprüngliche, nicht druckbelastete Volumen nach Unterschreiten des Druckschwellenwertes D wieder weitestgehend einstellt. Die erfindungsgemäße Gefäßprothese zeichnet sich dabei vor allem durch das besondere Material aus, aus dem die Volumenkammer vollständig oder teilweise gefertigt ist. Dieses Material umfasst ein spezielles elastisches Garn, welches aus einem Kern aus Silikongarn, der mit einem Umwindegarn aus Polyethylenterephthalat (PET) umwunden ist, gebildet ist. Der Werkstoff Silikon hat dabei hervorragende elastische Eigenschaften und trägt dazu bei, dass sich das Volumen der Volumenkammer bei zunehmendem Druck vergrößert und bei abnehmendem Druck im gleichen Maße wieder verringert. Hierdurch weist die neuartige Gefäßprothese im Bereich physiologischer Blutdrücke eine mit der Aorta identische Compliance auf. Die gleichzeitige Verwendung von PET zur Umwindung des Silikongarns trägt dazu bei, dass sich das Volumen der Volumenkammer erst bei Blutdrücken, die oberhalb eines voreinstellbaren, mindestens 100 mm Hg betragenden Druckschwellenwertes D liegen, deutlich vergrößert und dass diese Volumenzunahme insgesamt begrenzt ist, so dass auch bei sehr hohen Blutdrücken eine vorgegebene maximale Volumenvergrößerung nicht überschritten wird. Aufgrund der hohen Elastizität und damit einhergehender Rückstellkräfte bei Dehnung des verwendeten elastischen Garns sind sämtliche Aufweitungen der Volumenkammer reversibel, wodurch die Eigenschaften natürlicher Blutgefäße in hervorragender Weise nachgebildet werden können.

Gemäß Patentanspruch 1 weist die Volumenkammer in einem Blutdruckbereich unterhalb eines mindestens 100 mm Hg betragenden Druckschwellenwertes D ein Druck-Dehnungsverhalten auf, welches im Wesentlichen dem Druck-Dehnungsverhalten eines natürlichen Blutgefäßes entspricht. Hierunter ist zu verstehen, dass sich das Volumen der Volumenkammer unterhalb des Druckschwellenwertes D über die zur Aufrechterhaltung der Windkesselfunktion erforderlichen minimalen Ausdehnungen hinaus nicht ändert. Insbesondere sind diese Ausdehnungen aufgrund der Elastizität des Materials reversibel.

Nach Implantation der erfindungsgemäßen Gefäßprothese weitet sich die Volumenkammer der Gefäßprothese durch den in der Aorta herrschenden diastolischen Blutdruck (unterer Blutdruckwert) zunächst auf den vorhandenen Aortendurchmesser auf. Im Rahmen der normalen Herzaktion mit Auswurf eines gewissen Blutvolumens (Schlagvolumen) steigt der Blutdruck in der Aorta bzw. der Gefäßprothese bis zum Erreichen des systolischen Drucks an (oberer Blutdruckwert). Dadurch dehnt sich die Gefäßprothese im Bereich der elastischen Volumenkammer aus und wandelt einen Teil der kinetischen Auswurfsenergie in Spannenergie um. Bei Erschlaffen des Herzens während der Diastole mit entsprechendem Blutdruckabfall gibt die im Bereich der Volumenkammer elastisch ausgebildete Gefäßprothese die gespeicherte Energie aufgrund der wirkenden Rückstellkraft wieder ab und wirft dabei das gespeicherte Blutvolumen aus. Dies entspricht der normalen Windkesselfunktion der Aorta. Somit kommt es nach Implantation der erfindungsgemäßen elastischen Gefäßprothese nicht zu den nach Implantation von steifen Gefäßprothesen auftretenden ungünstigen Veränderungen der Blutdruckverhältnisse mit Beschleunigung der Pulswellengeschwindigkeit und Anstieg von Blutdruckkurve und -amplitude sowie den damit verbundenen negativen Langzeitfolgen.

Die erfindungsgemäße Gefäßprothese ist jedoch nicht nur in der Lage, die natürliche Windkesselfunktion sehr gut nachzubilden. Sie hat darüber hinaus bei vorliegendem erhöhtem Blutdruck auch eine ausgeprägte blutdrucksenkende Wirkung, was ebenfalls auf die elastische Ausgestaltung der Volumenkammer der Gefäßprothese zurückzuführen ist. Aufgrund der spezifischen Volumenkapazitätseigenschaften kommt es dabei erfindungsgemäß erst bei erhöhtem Blutdruck zu einer Senkung, wohingegen ein normaler oder niedriger Blutdruck nicht weiter gesenkt wird. So ist es erfindungsgemäß vorgesehen, dass das Volumen der Volumenkammer unterhalb eines mindestens 100 mm Hg betragenden Druckschwellenwertes D im Wesentlichen unveränderlich bleibt und sich über die zur Aufrechterhaltung der Windkesselfunktion erforderlichen minimalen Ausdehnungen hinaus nicht ändert. Erst ab Überschreiten des Druckschwellenwertes D erfolgt eine wesentliche und weitestgehend reversible radiale Aufweitung. Der Druckschwellenwert D beträgt dabei mindestens 100 mm Hg, er kann auch höher liegen und beispielsweise mindestens 120 mm Hg betragen. Die druckabhängige Volumenvergrößerung der Volumenkammer bei Überschreiten des Druckschwellenwertes D kann 10 - 80 cm³ betragen, vorzugsweise 40 - 60 cm³. Eine obere voreinstellbare Grenze der Volumenvergrößerung wird dabei jeweils nicht überschritten. Diese Begrenzung in der Volumenzunahme wird im Wesentlichen durch die Umwindung des Silikongarns mit PET bewirkt. Zusätzlich spielen hierbei sowie bei der Voreinstellung des Druckschwellenwertes D und der möglichen Volumenzunahme den konkreten textilen Schlauch bestimmende Parameter wie Fadenfeinheit, Filamentzahl, Bindungsart etc. eine Rolle, was im Folgenden noch genauer ausgeführt wird.

Aufgrund dieser Eigenschaften ist die Anwendung der erfindungsgemäßen elastischen Gefäßprothese bei Patienten mit therapieresistenter arterieller Hypertonie, d. h. mit einem Bluthochdruck, der nicht auf eine medikamentöse Therapie anspricht, als interventionelle Langzeitalternative zur medikamentösen Therapie denkbar. Neben dem Einsatz als Aortenersatz könnte die erfindungsgemäße elastische Gefäßprothese beispielsweise während einer notwendig gewordenen Herzoperation von Patienten mit therapieresistenter Hypertonie zusätzlich in Form eines Aortenbypasses als dauerhaft blutdrucksenkende Maßnahmen implantiert werden. Dabei ist es grundsätzlich denkbar, die Implantation minimalinvasiv und damit bei entsprechender Indikation auch als primären Eingriff zur dauerhaften Blutdrucksenkung durchzuführen.

Die erfindungsgemäße Gefäßprothese hat damit in hohem Maße adaptive Eigenschaften derart, dass sie bei normalen Blutdrücken die natürliche Windkesselfunktion der natürlichen Gefäße sehr gut nachbildet und damit einen hervorragenden Ersatz für einen Abschnitt eines natürlichen Blutgefäßes darstellt, während sie bei hohen Blutdrücken durch eine erst dann einsetzende zusätzliche und deutliche Volumenzunahme gleichzeitig auch eine ausgeprägte blutdrucksenkende Wirkung hat. Aufgrund des verwendeten Materials sind dabei die deutlichen Volumenänderungen bei hohen Blutdrücken weitestgehend reversibel, so dass sich nach Unterschreiten des Druckschwellenwertes D das ursprüngliche, nicht druckbelastete Volumen der Volumenkammer wieder weitestgehend einstellt. Die erfindungsgemäße Gefäßprothese weist somit nicht nur eine gute Dehnbarkeit, sondern gleichzeitig auch eine hohe Elastizität auf.

Schließlich ist es auch denkbar, die erfindungsgemäße elastische Gefäßprothese als aortokoronaren Bypass einzusetzen. Ein operativ angelegter aortokoronarer Bypass überbrückt vorhandene Engstellen der Herzkranzgefäße, sogenannte Koronarstenosen, und leitet das Blut aus der Aorta hinter die Koronarstenose zum Herzmuskel, wodurch die Durchblutung des Herzmuskels gewährleistet wird. Üblicherweise werden dafür bislang körpereigene Venen aus dem Bein oder, aufgrund ihrer elastischen Eigenschaft, bevorzugt körpereigene Arterien, beispielsweise Brustwandarterie, verwendet. Versuche mit bislang bekannten künstlichen Gefäßprothesen als aortokoronares Bypassmaterial waren aufgrund der fehlenden Elastizität und der damit verbundenen hohen Verschlussrate der kleinlumigen Gefäßprothesen erfolglos. Diese Einschränkung könnte mit der erfindungsgemäßen elastischen Gefäßprothese behoben werden, sodass die Entnahme von körpereigenen Venen am Bein bzw. Arterien an der Brustinnenwand während einer aortokoronaren Bypassoperation nicht mehr notwendig wäre. Damit könnten sowohl das Operationstrauma und die damit verbundenen Operationsrisiken (Nachblutungen, Wundheilungsstörungen und Infektionen im OP-Gebiet) minimiert als auch die Operationszeit deutlich verkürzt werden. Neben den direkten gesundheitlichen Vorteilen für den Patienten hätte dies auch positive gesundheitsökonomische Auswirkungen.

Grundsätzlich werden die speziellen Eigenschaften, d. h. die druckabhängige Volumenvergrößerung der Volumenkammer bei Überschreiten eines Druckschwellenwertes D und das Zurückkehren der Volumenkammer in den Ausgangszustand bei Unterschreiten des Druckschwellenwertes D sowie das bei Blutdrücken unterhalb des Druckschwellenwertes D vorliegende Druck-Dehnungsverhalten, welches dem eines natürlichen Gefäßes weitestgehend entspricht, im Wesentlichen durch die Materialkombination aus Silikon und PET in einem speziellen, für den medizinischen Einsatz geeigneten elastischen Garn bewirkt. Der konkrete Volumen-Druck-Verlauf für eine gegebene Gefäßprothese kann dabei durch zahlreiche Parameter eingestellt werden. Hierzu zählen Variationen in der Beschaffenheit und Ausgestaltung des elastischen Garns, insbesondere der Ausgangsmaterialien Silikongarn und PET-Umwindegarn, und der darüber hinaus zur Herstellung des textilen Schlauchs verwendeten PET-Garne, aber auch Variationen in der Ausgestaltung des unter Verwendung des elastischen Garns gefertigten, insbesondere gewebten, textilen Schlauchs.

Gemäß einer Ausgestaltung der Erfindung kann das den Kern des elastischen Garns bildende Silikongarn beispielsweise als Monofil aus Silikon einer Shorehärte von 30 bis 70, bevorzugt 40 bis 60 gebildet sein.

Das aus Silikongarn und PET-Umwindegarn gebildete elastische Garn kann eine Fadenfeinheit von 100 bis 3000 dtex, bevorzugt 200 bis 2000 dtex, aufweisen.

Das PET-Umwindegarn kann eine Fadenfeinheit von 30 bis 150 dtex, bevorzugt 50 bis 110 dtex aufweisen. Dabei kann das Silikongarn einfach oder mehrfach mit dem PET-Umwindegarn umwunden sein, wobei bei einer mehrfachen Umwindung mehrere Lagen PET-Umwindegarn auf dem Silikongarn zu liegen kommen. Bei einer zweifachen Umwindung liegt das PET-Umwindegarn entsprechend in zwei Lagen auf dem Silikongarn. Durch die Auswahl der Feinheit des PET-Umwindegarns ebenso wie durch die Anzahl und Dichte der Umwindungen und die Steigung, mit welcher das PET-Umwindegarn um das Silikongarn gewunden ist, kann das Druck-Dehnungsverhalten des elastischen Garns beeinflusst werden.

Eine Ausgestaltung der Erfindung sieht vor, dass die Gefäßprothese als Gewebeschlauch, insbesondere als nahtloser Gewebeschlauch, aus Kettfäden und Schussfäden ausgebildet ist, wobei die Schussfäden im Bereich der Volumenkammer aus dem elastischen Garn gebildet sind. Die Kettfäden können beispielsweise aus einem PET-Garn gebildet sein. Das die Kettfäden bildende PET-Garn kann eine Fadenfeinheit von 50 bis 300 dtex aufweisen und beispielsweise als Multifilamentgarn aus 20 bis 300 Filamenten ausgebildet sein. Dabei kann für die Zwecke der Erfindung als PET-Garn für die Kettfäden ein Glattgarn oder ein Texturgarn verwendet werden, für die Schussfäden kann in Bereichen angrenzend an die Volumenkammer, beispielsweise im Bereich der im Folgenden noch näher erläuterten Anschlussbereiche, ein Glattgarn oder ein Hochschrumpfgarn verwendet werden. Es können auch für unterschiedliche Abschnitte der Gefäßprothese unterschiedliche PET-Garne verwendet werden, so kann beispielsweise für die Grundkette ein PET-Glattgarn und für die Polkette ein PET-Texturgarn verwendet werden.

Hochschrumpfgarn hat die Eigenschaft, sich unter Wärmeeintrag zusammenzuziehen. Diese Eigenschaft kann insofern ausgenutzt werden, da bei Verwendung von Hochschrumpfgarn durch eine abschließende Thermofixierung eine Verdichtung des den textilen Schlauch bildenden Gewebes oder Gewirkes oder Gestrickes herbeigeführt werden kann.

Alternativ ist es auch denkbar, dass bei Ausbildung der Gefäßprothese als Gewebeschlauch sowohl die Kettfäden als auch die Schussfäden im Bereich der Volumenkammer aus dem elastischen Garn gebildet sind.

Für die Ausbildung des Gewebeschlauchs können unterschiedliche Gewebebindungen zugrunde gelegt werden. So kann der Gewebeschlauch beispielsweise in Köperbindung oder in Atlasbindung oder in Leinwandbindung oder in einer Abwandlung einer dieser Gewebebindungen gewebt sein. Grundsätzlich kann der Gewebeschlauch in einer einzigen Gewebebindung ausgeführt sein, der Gewebeschlauch kann jedoch auch Bereiche umfassen, die in unterschiedlichen Gewebebindungen ausgeführt sind. Durch die Auswahl der Gewebebindung kann unter anderem Einfluss genommen werden auf das Kraft-Dehnungsverhalten des Gewebeschlauchs.

Auch durch die Auswahl der Fadendichte, d. h. die Anzahl an Fäden pro cm, sowohl in Kettrichtung als auch in Schussrichtung kann das Kraft-Dehnungsverhalten des Gewebeschlauchs beeinflusst werden.

Zum Annähen der Gefäßprothese an die Enden eines natürlichen Blutgefäßes weist die Gefäßprothese an ihren beiden Enden Anschlussbereiche auf. Diese Anschlussbereiche können hinsichtlich Material und Elastizität gleichartig wie die Volumenkammer oder andersartig als die Volumenkammer ausgebildet sein.

So kann es vorgesehen sein, dass die Gefäßprothese an die Volumenkammer anschließende Anschlussbereiche zum Anschluss an das natürliche kardiovaskuläre System umfasst, wobei die Anschlussbereiche eine geringere Elastizität aufweisen als die Volumenkammer. Mit anderen Worten ist es bei dieser Ausführungsform der Erfindung vorgesehen, dass die Gefäßprothese Bereiche unterschiedlicher Elastizität aufweist, wobei ein zentraler, die Volumenkammer ausbildender Bereich eine höhere Elastizität aufweist als an die Volumenkammer beidseitig angrenzende Anschlussbereiche. Insbesondere können die Anschlussbereiche nicht-elastisch ausgebildet sein. Sie können beispielsweise als Gewebe ausgebildet sein, bei welchem sowohl die Kett- als auch die Schussfäden aus einem Multifilamentgarn aus PET gebildet sind. Anders als im Bereich der Volumenkammer kommt in den angrenzenden Anschlussbereichen bei dieser Ausführungsvariante somit kein elastisches Garn zum Einsatz.

Alternativ ist es auch möglich, dass endseitige Abschnitte der Volumenkammer selbst als Anschlussbereiche fungieren. In diesem Fall kann die Gefäßprothese als Ganze aus einem einheitlichen Material gefertigt sein und eine im Wesentlichen einheitliche Elastizität aufweisen.

Die erfindungsgemäße Gefäßprothese kann gemäß einer Ausgestaltung im Bereich der Anschlussbereiche mehrlagig, insbesondere doppellagig ausgeführt sein. Hierdurch wird die Nahtausreißkraft vergrößert, d. h. die Wahrscheinlichkeit, dass es zu einem Ausreißen der die Gefäßprothese mit dem natürlichen Gefäßsystem verbindenden Naht kommt, kann wesentlich herabgesetzt werden. Eine Mehrlagigkeit kann beispielsweise durch Umstülpen bzw. Umschlagen und gegebenenfalls Vernähen des textilen Schlauchs im Bereich der Anschlussbereiche erreicht werden.

Gemäß einer Ausgestaltung der Erfindung kann die Gefäßprothese hantelförmig ausgebildet sein, derart, dass sie in einem nicht druckbelasteten Zustand im Bereich der Anschlussbereiche gegenüber dem zentralen Bereich der Volumenkammer konisch aufgeweitet ist. Mit anderen Worten weist die Gefäßprothese bei dieser Ausgestaltung im Bereich der Volumenkammer in einem nicht druckbelasteten Zustand einen geringeren Durchmesser auf als im Bereich der Anschlussbereiche, deren Durchmesser sich ausgehend von dem Durchmesser der Volumenkammer zu den Enden der Gefäßprothese hin konisch aufweitet. Der Durchmesser der Gefäßprothese kann dabei im Bereich der Anschlussbereiche um etwa ein Drittel größer sein als der Durchmesser der Volumenkammer in einem nicht druckbelasteten Zustand. Es hat sich gezeigt, dass die natürliche Compliance der Aorta durch eine derartige hantelförmige Ausgestaltung der Gefäßprothese besonders gut nachgebildet werden kann.

Die Gefäßprothese kann eine Gesamtlänge von etwa 15 cm bis 25 cm aufweisen, wobei die Volumenkammer eine Länge von etwa 10 cm bis 15 cm aufweisen kann und die sich gegebenenfalls beiderseitig an die Volumenkammer anschließenden Anschlussbereiche jeweils eine Länge von etwa 3 cm bis 8 cm aufweisen können. Durch ein oben beschriebenes Umschlagen im Bereich der Anschlussbereiche kann die Länge der Anschlussbereiche dabei in einem gewissen Bereich variiert und an die Gegebenheiten angepasst werden. Der Durchmesser der Gefäßprothese im nicht druckbelasteten Zustand kann im zentralen Bereich der Volumenkammer etwa 15 mm bis 20 mm betragen. Bei einer Ausgestaltung der Gefäßprothese mit konisch aufgeweiteten Anschlussbereichen kann sich der Durchmesser bis auf etwa 25 mm bis 33 mm aufweiten.

Zur Gewährleistung der Anfangsdichtigkeit kann die Gefäßprothese mit vernetzter Gelatine imprägniert sein oder mit einem synthetischen Polymer, beispielsweise Silikon, beschichtet sein.

Schließlich betrifft die Erfindung gemäß Patentanspruch 16 auch ein Verfahren zum Herstellen einer erfindungsgemäßen Gefäßprothese. Das Verfahren umfasst dabei die folgenden Schritte:
- Bereitstellen von Schuss- und Kettfäden, wobei mindestens im Bereich der Volumenkammer die Schussfäden aus dem elastischen Garn gebildet sind,
- Schären von Grund- und Polkette,
- Weben des textilen Schlauches auf einer Schiffchen-Bandwebmaschine,
- Thermofixieren des gewebten textilen Schlauches,
- Waschen der Gefäßprothese zur Entfernung von Avivagen.

Zusammenfassend weist die erfindungsgemäße Gefäßprothese gegenüber bekannten Gefäßprothesen den Vorteil eines sehr einfachen Aufbaus auf, da sie die erforderlichen Eigenschaften vornehmlich aus dem Material bezieht, aus dem sie hergestellt ist. Auf einen aufwendigen und defektanfälligen Aufbau der Gefäßprothese, beispielsweise mit die Volumenkammer äußerlich umgebenden Anpassungsmitteln aus einem Rahmen und einem eingespannten Federkörper, kann somit verzichtet werden.

Die Elastizität und Volumenkapazität (Compliance) der erfindungsgemäßen elastischen textilen Gefäßprothese entspricht denen der natürlichen Aorta. Somit wird das bei steifen Gefäßprothesen vorhandene Compliance-Missverhältnis zwischen Gefäßprothese und Aorta beseitigt, was sich bei der bestimmungsgemäßen Langzeitanwendung günstig auf Herz, Aorta und die Gefäßprothese selbst auswirkt. Hierdurch können die oben beschriebenen negativen Folgen für Herz, Aorta und Gefäßprothese nach Implantation der Gefäßprothese vermieden werden.

Die erfindungsgemäße Gefäßprothese eignet sich in besonderem Maße für den Ersatz und die Überbrückung erkrankter Abschnitte der Aorta, aber auch größerer, mittlerer und kleinerer Arterien sowie als Gefäßprothese für aortokoronare Bypässe.

Im Folgenden wird die Erfindung anhand von Ausführungsbeispielen und unter Bezugnahme auf die beigefügten schematischen Zeichnungen näher erläutert.

Es zeigen:
- Figur 1:: eine erste Ausführungsform der erfindungsgemäßen elastischen Gefäßprothese in schematischer perspektivischer Darstellung;
- Figur 2:: ein Ausschnitt einer Darstellung einer zweiten Ausführungsform der erfindungsgemäßen elastischen Gefäßprothese;
- Figur 3:: ein Ausschnitt einer Darstellung einer dritten Ausführungsform der erfindungsgemäßen elastischen Gefäßprothese;
- Figur 4:: ein Ausschnitt einer Darstellung einer vierten Ausführungsform der erfindungsgemäßen elastischen Gefäßprothese;
- Figur 5:: ein Ausschnitt einer Darstellung einer fünften Ausführungsform der erfindungsgemäßen elastischen Gefäßprothese.

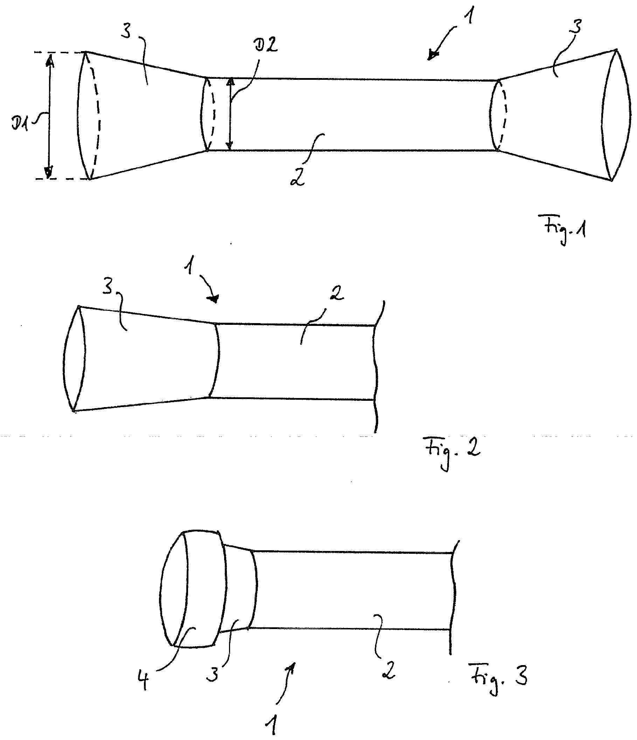

Figur 1 zeigt in schematischer perspektivischer Darstellung eine erste Ausführungsform einer im Ganzen mit 1 bezeichneten erfindungsgemäßen Gefäßprothese. Die Gefäßprothese 1 umfasst eine Volumenkammer 2 sowie an die Volumenkammer 2 anschließende Anschlussbereiche 3 zum Anschluss der Gefäßprothese 1 an ein hier nicht dargestelltes natürliches kardiovaskuläres System. Die Gefäßprothese 1 ist als nahtloser, textiler Gewebeschlauch aus Kettfäden und Schussfäden ausgebildet, wobei die Schussfäden im Bereich der Volumenkammer 2 aus einem hochelastischen Garn gebildet sind, welches einen Kern aus Silikongarn aufweist, der mit einem Garn aus Polyethylenterephthalat-Umwindegarn einfach umwunden ist. Das elastische Garn ist ein für medizinische Zwecke geeignetes Garn. Die Kettfäden im Bereich der Volumenkammer 2 sind aus einem für medizinische Zwecke geeigneten PET-Multifilamentgarn gebildet. Die endseitigen Anschlussbereiche 3 der Gefäßprothese 1 sind als nicht-elastisches Gewebe ausgebildet, wobei hier sowohl die Kettfäden als auch die Schussfäden aus einem PET-Multifilamentgarn gebildet sind.

Die Gefäßprothese 1 gemäß Figur 1 ist hantelförmig ausgebildet, d. h. in einem in Figur 1 dargestellten nicht druckbelasteten Zustand ist sie im Bereich der Anschlussbereiche 3 gegenüber dem zentralen Bereich der Volumenkammer 2 konisch aufgeweitet. Dabei ist der maximale Durchmesser D1 im Bereich der Anschlussbereiche 3 um etwa ein Drittel größer als der Durchmesser D2 im Bereich der Volumenkammer 2, was in der Figur 1 lediglich schematisch und nicht maßstabsgerecht dargestellt ist.

Figur 2 zeigt eine zweite Ausführungsform einer erfindungsgemäßen Gefäßprothese 1, wobei hier und ebenso in den Figuren 3 bis 5 jeweils nur eine Hälfte der symmetrisch aufgebauten Gefäßprothese 1 dargestellt ist. Das Ausführungsbeispiel der Figur 2 unterscheidet sich von dem der Figur 1 lediglich dadurch, dass die konische Aufweitung im Bereich der Anschlussbereiche 3 weniger stark ausgeprägt ist. In dem hier dargestellten Beispiel ist der maximale Durchmesser im Bereich der Anschlussbereiche um maximal ein Viertel größer als der Durchmesser im Bereich der Volumenkammer in einem nicht druckbelasteten Zustand derselben.

Figur 3 zeigt eine Ausführungsform der Gefäßprothese 1, welche im Bereich der Anschlussbereiche 3 doppellagig ausgeführt ist. Dazu ist der Anschlussbereich 3 der Gefäßprothesen 1 gemäß Figur 1 in einem Endbereich 4 nach außen umgestülpt, wobei zur Fixierung die außenliegende Lage in nicht näher dargestellter Art und Weise mit der innenliegenden Lage vernäht ist. Wird eine derartige Gefäßprothese 1 im Bereich des doppellagigen Anschlussbereichs mit einem natürlichen Blutgefäß vernäht, kann durch die Doppellagigkeit einem Ausreißen der Naht entgegengewirkt werden.

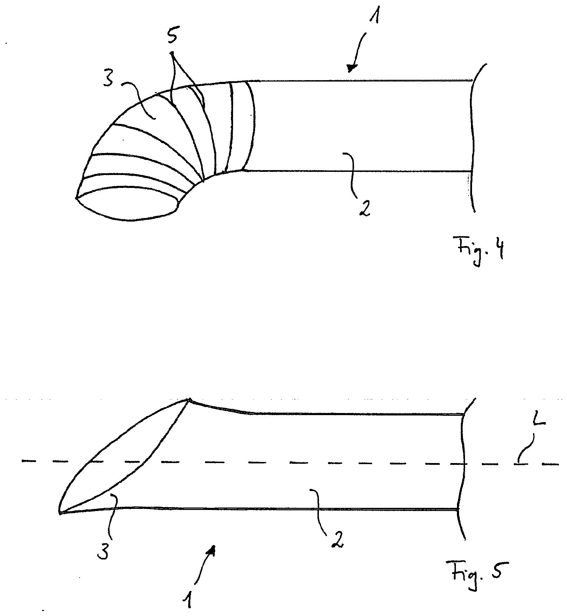

Figur 4 zeigt eine Ausführungsform der Gefäßprothese 1, bei der der Durchmesser im Bereich der Anschlussbereiche 3 im Wesentlichen dem Durchmesser im Bereich der Volumenkammer 2 im nicht druckbelasteten Zustand entspricht. Gleichzeitig weist die Gefäßprothese 1 im Bereich der Anschlussbereiche 3 in Umfangsrichtung eine Plissierung 5 auf. Die Plissierung 5 kann wendelförmig oder in Form von geschlossenen Ringen ausgebildet sein und verleiht der Gefäßprothese im Bereich der Anschlussbereiche 3 eine hohe Flexibilität. Insbesondere kann ein Abknicken der Gefäßprothese 1 im Bereich des Anschlusses an ein natürliches Blutgefäß hierdurch verhindert werden.

Figur 5 zeigt eine weitere Ausgestaltung der erfindungsgemäßen Gefäßprothese 1, bei welcher endseitige Abschnitte der Volumenkammer 2 selbst als Anschlussbereiche 3 fungieren. In diesem Fall ist die Gefäßprothese 1 als Ganze aus einem einheitlichen, elastischen Material gewebt, wobei für die Schussfäden das elastische Garn aus Silikongarn mit PET-Umwindegarn und für die Kettfäden ein PET-Multifilamentgarn eingesetzt wird. Die Gefäßprothese 1 weist im druckunbelasteten Zustand über ihre gesamte Länge einen im Wesentlichen einheitlichen Durchmesser auf. Im Bereich der Anschlussbereiche 3 ist die Volumenkammer 2 abgeschrägt ausgebildet. Der Winkel der Abschrägung beträgt gegenüber der Längsachse L der Gefäßprothese 1 etwa 45°. Hierdurch eignet sich eine solche Gefäßprothese 1 besonders für die Implantation als Bypass. Eine derartige Gefäßprothese 1 kann aus einem entsprechenden textilen Endlosschlauch durch einfaches Ablängen und Abschrägen im Bereich der Schnittkanten hergestellt werden. Das Abschrägen kann dabei beispielsweise durch Laserschneiden erfolgen. Hierdurch können besonders saubere, nicht ausfransende Schnittkanten realisiert werden. Eine derartig ausgebildete Gefäßprothese weist einen besonders einfachen Aufbau auf und ist besonders unaufwändig herzustellen.

## Patentansprüche

1. Gefäßprothese (1) zum Anschluss an ein natürliches kardiovaskuläres System, umfassend eine Volumenkammer (2), wobei die Volumenkammer (2) in einem Blutdruckbereich unterhalb eines mindestens 100 mm Hg betragenden Druckschwellenwertes D ein Druck-Dehnungsverhalten aufweist, welches im Wesentlichen dem Druck-Dehnungsverhalten eines natürlichen Blutgefäßes entspricht, während sich das Volumen der Volumenkammer (2) in einem Blutdruckbereich oberhalb des Druckschwellenwertes D druckabhängig um mindestens 10 cm³ vergrößert, **dadurch gekennzeichnet, dass** die Gefäßprothese (1) als textiler Schlauch ausgebildet ist, wobei der textile Schlauch im Bereich der Volumenkammer (2) ein elastisches Garn umfasst, welches einen Kern aus Silikongarn aufweist, der mit einem Garn aus Polyethylenterephthalat (PET) umwunden ist.

2. Gefäßprothese (1) nach Anspruch 1, **dadurch gekennzeichnet, dass** der Kern des elastischen Garns ein hochelastisches Silikongarn ist.

3. Gefäßprothese (1) nach Anspruch 1 oder 2, **dadurch gekennzeichnet, dass** das Silikongarn als Monofil aus Silikon mit einer Shorehärte von 30 bis 70, vorzugsweise 40 bis 60, gebildet ist.

4. Gefäßprothese (1) nach einem der Ansprüche 1 bis 3, **dadurch gekennzeichnet, dass** das elastische Garn eine Fadenfeinheit von 100 bis 3000 dtex, vorzugsweise 200 bis 2000 dtex, aufweist.

5. Gefäßprothese (1) nach einem der Ansprüche 1 bis 4, **dadurch gekennzeichnet, dass** die Gefäßprothese (1) als Gewebeschlauch aus Kettfäden und Schussfäden ausgebildet ist, wobei die Schussfäden im Bereich der Volumenkammer (2) aus dem elastischen Garn gebildet sind.

6. Gefäßprothese (1) nach Anspruch 5, **dadurch gekennzeichnet, dass** die Kettfäden aus einem PET-Garn gebildet sind.

7. Gefäßprothese (1) nach Anspruch 6, **dadurch gekennzeichnet, dass** das die Kettfäden bildende PET-Garn eine Fadenfeinheit von 50 bis 300 dtex aufweist.

8. Gefäßprothese nach Anspruch 6 oder 7, **dadurch gekennzeichnet, dass** das die Kettfäden bildende PET-Garn als Multifilamentgarn aus 20 bis 300 Filamenten ausgebildet ist.

9. Gefäßprothese (1) nach einem der Ansprüche 6 bis 8, **dadurch gekennzeichnet, dass** das die Kettfäden bildende PET-Garn als Glattgarn oder als Texturgarn ausgebildet ist.

10. Gefäßprothese (1) nach einem der Ansprüche 5 bis 9, **dadurch gekennzeichnet, dass** der Gewebeschlauch in Köperbindung oder Atlasbindung oder Leinwandbindung gewebt ist.

11. Gefäßprothese (1) nach einem der Ansprüche 1 bis 10, **dadurch gekennzeichnet, dass** die Gefäßprothese (1) an die Volumenkammer (2) anschließende Anschlussbereiche (3) zum Anschluss an das natürliche kardiovaskuläre System umfasst, wobei die Anschlussbereiche (3) eine geringere Elastizität aufweisen als die Volumenkammer (2).

12. Gefäßprothese (1) nach Anspruch 11, **dadurch gekennzeichnet, dass** die Anschlussbereiche (3) als Gewebe ausgebildet sind und als Multifilamentgarn ausgebildete Kett- und Schussfäden aus PET umfassen, wobei die Schussfäden als Glattgarn oder als Hochschrumpfgarn ausgebildet sind.

13. Gefäßprothese (1) nach einem der Ansprüche 1 bis 10, **dadurch gekennzeichnet, dass** endseitige Abschnitte der Volumenkammer als Anschlussbereiche zum Anschluss an das natürliche kardiovaskuläre System fungieren, wobei die Gefäßprothese im Bereich der Volumenkammer einschließlich der Anschlussbereiche eine einheitliche Elastizität aufweist.

14. Gefäßprothese (1) nach einem der Ansprüche 11 bis 13, **dadurch gekennzeichnet, dass** die Gefäßprothese (1) im Bereich der Anschlussbereiche (3) doppellagig ausgeführt ist.

15. Gefäßprothese (1) nach einem der Ansprüche 11 bis 14, **dadurch gekennzeichnet, dass** die Gefäßprothese (1) hantelförmig ausgebildet ist, derart, dass sie in einem nicht druckbelasteten Zustand im Bereich der Anschlussbereiche (3) gegenüber dem zentralen Bereich der Volumenkammer (2) konisch aufgeweitet ist.

16. Verfahren zur Herstellung einer Gefäßprothese (1) nach einem der Ansprüche 1 bis 15, umfassend die folgenden Schritte:
- Bereitstellen von Schuss- und Kettfäden, wobei mindestens im Bereich der Volumenkammer die Schussfäden aus dem elastischen Garn gebildet sind,
- Schären von Grund- und Polkette,
- Weben des textilen Schlauches auf einer Schiffchen-Bandwebmaschine,
- Thermofixieren des gewebten textilen Schlauches
- Waschen der Gefäßprothese (1) zur Entfernung von Avivagen.

## Claims

1. Vascular prosthesis (1) for connection to a natural cardiovascular system, comprising a volume chamber (2), wherein the volume chamber (2) has, in a blood pressure range below a pressure threshold value D of at least 100 mm Hg, a pressure-expansion behaviour substantially corresponding to the pressure-expansion behaviour of a natural blood vessel, while the volume of the volume chamber (2), depending on the pressure, increases by at least 10 cm3 in a blood pressure range above the pressure threshold value D, **characterised in that** the vascular prosthesis (1) is a textile tube, wherein the textile tube includes, in the region of the volume chamber (2), an elastic yarn having a core made from silicone yarn around which a yarn made from polyethylene terephthalate (PET) is wrapped.

2. Vascular prosthesis (1) according to claim 1, **characterised in that** the core of the elastic yarn is a highly elastic silicone yarn.

3. Vascular prosthesis (1) according to claim 1 or 2, **characterised in that** silicone yarn is formed as a monofilament of silicone with a Shore hardness of 30 to 70, preferably 40 to 60.

4. Vascular prosthesis (1) according to any one of claims 1 to 3, **characterised in that** the elastic yarn has a thread count of 100 to 3000 dtex, preferably 200 to 2000 dtex.

5. Vascular prosthesis (1) according to any one of the claims 1 to 4, **characterised in that** the vascular prosthesis (1) is a woven-fabric tube made of warp threads and weft threads, wherein the weft threads are formed from the elastic yarn in the region of the volume chamber (2).

6. Vascular prosthesis (1) according to claim 5, **characterised in that** the warp threads are formed from a PET yarn.

7. Vascular prosthesis (1) according to claim 6, **characterised in that** the PET yarn forming the warp threads has a thread count of 50 to 300.

8. Vascular prosthesis according to claim 6 or 7, **characterised in that** the PET yarn forming the warp threads is multifilament yarn with 20 to 300 filaments.

9. Vascular prosthesis (1) according to any one of claims 6 to 8, **characterised in that** the PET yarn forming the warp threads is smooth yarn or textured yarn.

10. Vascular prosthesis (1) according to any one of claims 5 to 9, **characterised in that** the woven-fabric tube is woven in a twill weave or satin weave or plain weave.

11. Vascular prosthesis (1) according to any one of claims 1 to 10, **characterised in that** the vascular prosthesis (1) comprises connection areas (2) adjoining the volume chamber (2) for connection to the natural cardiovascular system, wherein the connection areas (3) have a lower elasticity than the volume chamber (2).

12. Vascular prosthesis (1) according to claim 11, **characterised in that** the connection areas (3) are woven fabric and comprise PET warp threads and weft threads as multifilament yarn, wherein the weft threads are smooth yarn or highly heat-shrinking yarn.

13. Vascular prosthesis (1) according to any one of claims 1 to 10, **characterised in that** end sections of the volume chamber function as connection areas for connection to the natural cardiovascular system, wherein the vascular prosthesis has a uniform elasticity in the region of the volume chamber including the connection areas.

14. Vascular prosthesis (1) according to any one of claims 11 to 13, **characterised in that** the vascular prosthesis (1) is produced double-layered in the region of the connection areas (3).

15. Vascular prosthesis (1) according to any one of claims 11 to 14, **characterised in that** the vascular prosthesis (1) is dumbbell-shaped, such that, in a non-pressure loaded state, it is conically widened in the region of the connection areas (3) compared with the central region of the volume chamber (2).

16. Method for manufacturing a vascular prosthesis (1) according to any one of claims 1 to 15, comprising the following steps:
- providing weft and warp threads wherein, at least in the region of the volume chamber, the weft threads are formed from the elastic yarn,
- warping of base warp and pile warp,
- weaving of the textile tube on a shuttle ribbon loom,
- heat setting of the woven textile tube,
- washing of the vascular prosthesis (1) in order to remove finishing agents.

## Revendications

1. Prothèse vasculaire (1) pour le raccordement à un système cardiovasculaire naturel, comprenant une chambre volumique (2), dans laquelle la chambre volumique (2) présente, dans une plage de pression artérielle inférieure à une valeur seuil de pression (D) s'élevant au moins à 100 mm Hg, un comportement de dilatation à la pression qui correspond sensiblement au comportement de dilatation à la pression d'un vaisseau sanguin naturel, tandis que le volume de la chambre volumique (2) augmente, dans une plage de pression artérielle supérieure à la valeur seuil de pression D, d'au moins 10 cm³ en fonction de la pression, **caractérisée en ce que** la prothèse vasculaire (1) est réalisée comme un tube textile, dans laquelle le tube textile comprend dans la zone de la chambre volumique (2) un fil élastique qui présente une âme en fil de silicone qui est enroulé avec un fil de polyéthylène téréphtalate (PET).

2. Prothèse vasculaire (1) selon la revendication 1, **caractérisée en ce que** l'âme du fil élastique est un fil de silicone hautement élastique.

3. Prothèse vasculaire (1) selon la revendication 1 ou 2, **caractérisée en ce que** le fil de silicone est formé comme un monofilament de silicone présentant une dureté Shore de 30 à 70, de préférence de 40 à 60.

4. Prothèse vasculaire (1) selon l'une quelconque des revendications 1 à 3, **caractérisée en ce que** le fil élastique présente une finesse de 100 à 3 000 dtex, de préférence de 200 à 2 000 dtex.

5. Prothèse vasculaire (1) selon l'une quelconque des revendications 1 à 4, **caractérisée en ce que** la prothèse vasculaire (1) est réalisée comme un tube de tissu en fils de chaîne et fils de trame, dans laquelle les fils de trame sont formés à partir du fil élastique dans la zone de la chambre volumique (2).

6. Prothèse vasculaire (1) selon la revendication 5, **caractérisée en ce que** les fils de chaîne sont formés à partir d'un fil de PET.

7. Prothèse vasculaire (1) selon la revendication 6, **caractérisée en ce que** le fil de PET formant les fils de chaîne présente une finesse de 50 à 300 dtex.

8. Prothèse vasculaire (1) selon l'une quelconque des revendications 6 ou 7, **caractérisée en ce que** le fil de PET formant les fils de chaîne est réalisé comme un fil à multifilaments de 20 à 300 filaments.

9. Prothèse vasculaire (1) selon l'une quelconque des revendications 6 à 8, **caractérisée en ce que** le fil de PET formant les fils de chaîne est constitué comme un fil plat ou comme un fil texturé.

10. Prothèse vasculaire (1) selon l'une quelconque des revendications 5 à 9, **caractérisée en ce que** le tube de tissu est tissé en sergé ou en satin ou en toile.

11. Prothèse vasculaire (1) selon l'une quelconque des revendications 1 à 10, **caractérisée en ce que** la prothèse vasculaire (1) comprend des zones de raccordement (3) adjacentes à la chambre volumique (2) pour le raccordement au système cardiovasculaire naturel, dans laquelle les zones de raccordement (3) présentent une élasticité plus réduite que la chambre volumique (2).

12. Prothèse vasculaire (1) selon la revendication 11, **caractérisée en ce que** les zones de raccordement (3) sont réalisées comme un tissu et comprennent des fils de chaîne et de trame en PET réalisés comme un fil à multifilaments, dans laquelle les fils de trame sont réalisés comme un fil plat ou comme un fil cloqué.

13. Prothèse vasculaire (1) selon l'une quelconque des revendications 1 à 10, **caractérisée en ce que** des segments terminaux de la chambre volumique se présentent comme des zones de raccordement pour le raccordement au système cardiovasculaire naturel, dans laquelle la prothèse vasculaire présente, dans la zone de la chambre volumique incluant les zones de raccordement, une élasticité uniforme.

14. Prothèse vasculaire (1) selon l'une quelconque des revendications 11 à 13, **caractérisée en ce que** la prothèse vasculaire (1) est conçue à deux couches dans la zone des zones de raccordement (3).

15. Prothèse vasculaire (1) selon l'une quelconque des revendications 11 à 14, **caractérisée en ce que** la prothèse vasculaire (1) est réalisée en forme d'haltère, de telle sorte qu'elle s'élargisse de façon conique dans un état non soumis à la pression dans la zone des zones de raccordement (3) par rapport à la zone centrale de la chambre volumique (2).

16. Procédé de fabrication d'une prothèse vasculaire (1) selon l'une quelconque des revendications 1 à 15, comprenant les étapes suivantes :
- mise à disposition de fils de trame et de chaîne, dans lequel au moins dans la zone de la chambre volumique, les fils de trame sont formés à partir du fil élastique,
- ourdissage de la chaîne de fond et de la chaîne poil,
- tissage du tube textile sur un métier à tisser ruban à navettes,
- thermofixation du tube textile tissé
- lavage de la prothèse vasculaire (1) pour éliminer les avivages.
